# EUROPEAN PATENT SPECIFICATION

(11) **EP 1 137 407 B1**
(45) Date of publication and mention of the grant of the patent: **18.02.2004**
(21) Application number: 99905124.6
(22) Date of filing: 04.03.1999
(51) Int. Cl.: A61K 31/13, A61K 47/00, A61K 31/78, A61P 27/02

(54) **OPHTHALMIC FORMULATION COMPRISING A BETA BLOCKER AND CARBOPOL**
BETABLOCKER UND CARBOPOL ENTHALTENDE OPHTALMISCHE ZUSAMMENSETZUNG
FORMULATION OPHTALMIQUE COMPRENANT UN BETA-BLOQUANT ET DU CARBOPOL

(43) Date of publication of application: 04.10.2001
(73) Proprietor: Khamar, Bakulesh Mafatlal, Ahmedabad 380 006 (IN)
(72) Inventor: Khamar, Bakulesh Mafatlal, Ahmedabad 380 006 (IN)
(74) Representative: Towler, Philip Dean
(86) International application number: PCT/IB1999/000378
(87) International publication number: WO 2000/035439

(56) References cited:
- EP-A1- 0 227 494
- EP-A1- 0 582 321
- EP-A2- 0 332 147
- CHEMICAL ABSTRACTS, vol. 124, no. 26, 24 June 1996, Columbus, Ohio, US; abstract no. 352524C, DICKSTEIN ET AL.: 'Comparison of the effects of aqueous and gellan ophthalmic thimolol on peak exercise performance in middle-aged men' page 662; column 1; XP002947853 & ANM. J. OPHTHALMOL., vol. 121, no. 4, 1996, (ENG), pages 367 - 371

## Description

The present invention relates to ophthalmic formulations of Beta-blockers with improved efficacy and tolerance. Beta-blockers are used as topical ophthalmic preparations for reducing intraocular pressure (I.O.P.).

The present invention is directed formulations containing Beta-blockers in such a way so that pressure lowering effects of Beta-blockers are improved. Beta-blockers are required to be used for a long time for reduction in I.O.P. Their prolonged use is associated with instability of tear film leading to dry eye. The present invention is also directed to formulations containing Beta-blockers in such a way so that tear film is stabilized.

Beta-blockers are known to reduce I.O.P. mainly by reduction in aqueous secretion. This reduction in aqueous secretion is dose dependent. However, increasing the dosage beyond a point does not improve its capacity to reduce I.O.P. For timolol, levobunolol and Betaxalol it is achieved at 0.5% concentration for carteolol it is 1% and for metipranalol it is 0.3%. Increasing concentration beyond this does not result in further reduction in I.O.P.

The attempts made to improve its efficacy are not successful. In clinical situation when further reduction in I.O.P. is desired another drug like, Pilocarpine, Dipivefrin hydrochloride, Dorzdamide, Brimonidine, Latanoprost, etc. is added to it.

The formulations of Beta-blockers used are usually aqueous in nature. However, some compositions which undergo liquid-gel phase transition are also known: EP0227494 discloses compositions comprising 0.34 or 0.65% timolol maleate and Gelrite® (a gel forming substance derived from gellan gum); Kenneth et al. (Chemical Abstracts (1996) 124, 26, 352524c) describes a 0.5% timolol *gellan* suspension that forms a gel upon application to the conjunctiva.

There are sustained release preparations available for Beta-blockers. The formulation of pilocarpine in sustained release preparation is required to be doubled i.e. for I.O.P. reduction as much as 2% pilocarpine solution, 4% pilocarpine gel is required. Betaxolol is available as Betopitic-s and of timolol is Timoptic-XE. In both formulations vehicle used are different. With this it is possible to reduce concentration of Betaxolol used, but it is not possible to improve effect on I.O.P. Similarly, it is possible to reduce frequency of administration from twice a day to once a day with timoptic-XE. However, pressure lowering effect remains same. The formulations made with hydroxyl propyl methyl cellulose are found to be of no advantage compared to aqueous formulation.

Similarly, sustained release preparation of pilocarpine (Pilopine-HS gel) is also available. It contains Carbopol as a vehicle. The duration of action is prolonged but pressure reducing effect is reduced. To get the pressure lowering effect as much as aqueous solution, concentration of pilocarpine in sustained release preparation is required to be doubled i.e. for I.O.P. reduction as much as 2% pilocarpine solution, 4% pilocarpine gel is required.

The objective of present invention is to provide formulation of Beta-blockers with improved efficacy.

The further objective of present invention is to provide formulation of Beta-blocker which stabilizes the tear film.

The further objective ofpresent invention is to provide a formulation of Beta-blockers which is effective after longer period of storage.

The further objective of present invention is it minimize/eliminate Beta-blocker entering systemic circulation.

The further objective of present invention is to increase compliance by reduction/elimination of side effects of Beta-blockers.

The further objective of present invention is to provide formulation in concentration which is known to provide maximum I.O.P. lowering effect in a conventional aqueous formulation.

The present invention relates to an aqueous ophthalmic preparation comprising a Beta-blocker and a gel forming substance and optionally one or more physiologically acceptable excipient, buffer and preservative, characterised in that the gel forming substance is carbopol.

Beta-blockers described above can be timolol 0.5%, Betaxolol 0.5%, Levobunolol 0.5%, Carteolol 1.0%, metipranolol 0.3% or any other Beta-blocker which can reduce I.O.P. in a therapeutic concentration.

Carbopol can be carbopol 940, 932, 970 or others which form gel in aqueous solution. The concentration of carbopol in final formulation can be from 0.5% to 5%.

The buffer can be any used in topical ophthalmic preparation e.g. dibasic sodium phosphate, sodium phosphate monobasic.

The preservative can be EDTA, Benzyloconium chloride, Cetrimide or any other which can be used in ophthalmic topical preparation in a recommended dosage.

pH is usually acidic and needs to be adjusted by sodium hydroxide. Preferably, the pH of the formulation is 6.0 to 8.0, more preferably 6.5 to 7.5.

In a further embodiment of the invention, there is provided a process for preparing an ophthalmic preparation comprising the steps of:
i)
   a. making an aqueous solution of Beta-blocker
   b. making a gel of carbopol in a separate vessel;
ii) adding the aqueous solution of Beta-blocker into the prepared gel of carbopol while stirring slowly;
iii) adjusting the pH and volume before finally autoclaving and packaging.

Preferably, the process comprises the following steps:
1. The aqueous solution of Beta-blocker is made which contains acceptable excipients, buffers and preservative in distilled water. The pH of this solution is adjusted to 7.0 to 7.5.
2. In a separate vessel Carbopol is dissolved into water and stirred well till gel is formed. Preservatives and buffers are added to it gradually while stirring. The pH of solution is adjusted to pH 6.5 to 7.5.
3. Solution containing Beta-blocker as formulated in step 1 is gradually added to the gel as formed in step 2.
4. Volume is made up by adding distilled water as required.
5. pH is checked and adjusted as necessary to keep it in range of 7.0 ± 0.5.

Preferably, the final product is autoclaved before packaging.

### Example of formulation

I. Timolol 0.5%

| | |
|---|---|
| Timolol maleate | 0.72 gm equivalent to 0.5 gm of timolol |
| Benzylconium chloride | 0.0107 gm |
| Carbopol 940 | 2.0 gm |
| Sodium hyroxide to adjust pH | 6.5 to 7.5 |
| Water for injection | QS to make 100 ml. |

II. Betaxolol 0.5%

| | |
|---|---|
| Betaxolol hydrochloride | 0.56 gm equivalent to 0.5 gm ofBetaxolol |
| Benzylconium chloride | 0.01 gm |
| Di basic sodium phosphate | 0.05 gm |
| Sodium phosphate mono basic | 0.025 gm |
| Di sodium EDTA | 0.05 gm |
| Sodium chloride | 0.30 gm |
| Propylene glycol | 2.50 gm |
| Carbopol 940 | 2.00 gm |
| Water for injection | QS to make 100 ml of solution |

The pharmaceutical composition so manufactured is evaluated for stability and efficacy.

The pharmaceutical composition so manufactured is evaluated at different test conditions of temperature and humidity (45° C, 37° C at 80% relative humidity and ambient temperature), for time interval extending upto 12 months.

The samples of formulation were taken for study.

The formulation of timolol 0.5% made as described (new formulation) was evaluated in healthy volunteers as well as in eyes having raised intraocular pressure.

In a single dose paralleled study timolol 0.5% eye drops (conventional formulation) were instilled in one eye and new formulation was instilled in the other eye of 11 patients. Timolol eye drops caused drop in I.O.P. by 23.49% while new formulation caused drop in I.O.P. by 38.7%.

In a single dose cross over study (10 eyes) new formulation as well as conventional formulation (eye drops) were instilled in same eye on different days, but at the same time of day. It was found that reduction in I.O.P. with conventional formulation was 22.36% while that with new formulations was 37.7%.

Thus improved efficacy of new formulation is established in healthy volunteers.

Similarly, in glaucomatous eyes (14), both formulations (conventional and new) were evaluated. Even in glaucomatous eyes the reduction in I.O.P. noticed was much more than that seen with conventional formulation. With conventional formulation it was 33.35% while with new formulation drop in I.O.P. was 44.4%.

The effect on reduction in I.O.P. seen in glaucomatous eyes was further evaluated by long term application in 14 eyes. It was found that effect is maintained even on long term application. The drop in I.O.P. in glaucomatous eyes was 44.4% at 15 days, 43.6% at one month and 43.6% at three months interval.

Thus new formulation was found to have improved efficacy in glaucomatous eyes. This improved efficacy was found to persist even on long terms application.

Like eye drops of timolol, increasing concentration of timolol in new formulation from 0.5% to 1.0%, further drop in I.O.P. was not seen. However, this resulted in increase in duration of its action.

When other antiglaucoma drugs were added to therapy in persons using new formulation it was found to reduce I.O.P. further. This further reduction in I.O.P. was as good as seen with combination of antiglaucoma drugs with timolol eye drops.

Similarly, when formulation with other Beta-blockers like, Betaxolol were made as per process described in this invention it was also found to cause further drop in I.O.P. compared to conventional formulation.

Traditionally made viscous formulation for use as topical ophthalmic preparations are known to cause disturbances in vision. However, none of the person in whom new formulation were used complained of visual disturbances 5 minutes after instillation of new formulation.

## Claims

1. An aqueous ophthalmic preparation comprising a Beta-blocker and a gel forming substance and optionally one or more physiologically acceptable excipient, buffer and preservative,
**characterised in that** the gel forming substance is carbopol.

2. An ophthalmic preparation as claimed in claim 1 wherein the Beta-blocker is selected from the group consisting of timolol, betaxolol, carteolol and metipranalol.

3. A preparation as claimed in either claim 1 or claim 2 wherein the concentration of carbopol is 0.5% to 5%.

4. A preparation as claimed in any one of claims 1 to 3 wherein the pH of the formulation is 6.0-8.0.

5. A preparation as claimed in claim 4 wherein the pH of the formulation is 6.5-7.5.

6. A process for preparing an ophthalmic preparation of any one of claims 1 to 5 comprising the steps of:
i)
a. making an aqueous solution of Beta-blocker
b. making a gel of carbopol in a separate vessel;
ii) adding the aqueous solution of Beta-blocker into the prepared gel of carbopol while stirring slowly;
iii) adjusting the pH and volume before finally autoclaving and packaging.

7. A process as claimed in claim 6 wherein the formulation is autoclaved before packaging.

## Patentansprüche

1. Wäßrige opthalmische Zusammensetzung, umfassend einen Beta-Blocker und eine gelbildende Substanz und optional einen oder mehrere physiologisch annehmbare Trägerstoffe, Puffer und Konservierungsstoffe, **dadurch gekennzeichnet, daß** die gelbildende Substanz Carbopol ist.

2. Opthalmische Zusammensetzung nach Anspruch 1, wobei der Beta-Blocker ausgewählt ist aus der Gruppe bestehend aus Timolol, Betaxalol, Carteolol und Metipranalol.

3. Zusammensetzung nach Anspruch 1 oder Anspruch 2, wobei die Konzentration von Carbopol 0,5 % bis 5 % beträgt.

4. Zusammensetzung nach einem der Ansprüche 1 bis 3, wobei der pH-Wert der Formulierung 6,0 - 8,0 beträgt.

5. Zusammensetzung nach Anspruch 4, wobei der pH-Wert der Formulierung 6,5 - 7,5 beträgt.

6. Verfahren zur Herstellung einer opthalmischen Zusammensetzung nach einem der Ansprüche 1 bis 5, umfassend die Schritte:
i)
a. Herstellen einer wäßrigen Lösung eines Beta-Blockers
b. Herstellen eines Gels aus Carbopol in einem separaten Gefäß;
ii) Hinzufügen der wäßrigen Lösung des Betablockers zu dem hergestellten Gel aus Carbopol unter langsamem Rühren;
iii) Einstellen des pH-Werts und Volumens vor dem endgültigen Autoklavieren und Verpacken.

7. Verfahren nach Anspruch 6, wobei die Formulierung vor dem Verpacken autoklaviert wird.

## Revendications

1. Préparation ophtalmique aqueuse comprenant un bêtabloquant et une substance formant un gel et éventuellement un ou plusieurs excipients, tampons et conservateurs acceptables du point de vue physiologique, **caractérisée en ce que** la substance formant un gel est un carbopol.

2. Préparation ophtalmique selon la revendication 1, dans laquelle le bêtabloquant est choisi dans le groupe consistant en timolol, bêtaxolol, cartéolol et métipranolol.

3. Préparation selon la revendication 1 ou la revendication 2, dans laquelle la concentration de carbopol est de 0,5% à 5%.

4. Préparation selon l'une quelconque des revendications 1 à 3, dans laquelle le pH de la formulation est de 6,0 à 8,0.

5. Préparation selon la revendication 4, dans laquelle le pH de la formulation est de 6,5 à 7,5.

6. Procédé de fabrication d'une préparation ophtalmique selon l'une quelconque des revendications 1 à 5, comprenant les étapes de :
i)
a. préparation d'une solution aqueuse de bêtabloquant
b. préparation d'un gel de carbopol dans un récipient séparé ;
ii) addition de la solution aqueuse de bêtabloquant au gel préparé de carbopol avec agitation lente ;
iii) ajustement du pH et du volume avant un traitement final en autoclave et conditionnement.

7. Procédé selon la revendication 6, dans lequel la formulation est traitée en autoclave avant conditionnement.
